(19) Europäisches Patentamt
European Patent Office
Office européen des brevets

(11) Veröffentlichungsnummer: **0 490 188 A1**

(12) **EUROPÄISCHE PATENTANMELDUNG**

(21) Anmeldenummer: **91120533.4**

(22) Anmeldetag: **29.11.91**

(51) Int. Cl.[5]: **C07D 239/30**, C07D 239/34, C07D 239/70, C07D 401/12, C07D 403/12, C07D 405/12, A01N 43/54

(30) Priorität: **12.12.90 DE 4039630**

(43) Veröffentlichungstag der Anmeldung:
**17.06.92 Patentblatt 92/25**

(84) Benannte Vertragsstaaten:
**BE CH DE DK ES FR GB GR IT LI NL**

(71) Anmelder: **BAYER AG**

**W-5090 Leverkusen 1 Bayerwerk(DE)**

(72) Erfinder: **Kirsten, Rolf, Dr.**
**Carl-Langhans-Strasse 27**
**W-4019 Monheim(DE)**
Erfinder: **Wolf, Hilmar, Dr.**
**Zum Bräuhaus 14**
**W-4018 Langenfeld(DE)**
Erfinder: **Hänssler, Gerd, Dr.**
**Am Arenzberg 58a**
**W-5090 Leverkusen 3(DE)**
Erfinder: **Dehne, Heinz-Wilhelm, Dr.**
**Krischer Strasse 81**
**W-4019 Monheim(DE)**
Erfinder: **Dutzmann, Stefan, Dr.**
**Kosenberg 10**
**W-4010 Hilden(DE)**

(54) **Substituierte Pyrimidylamidoxime.**

(57) Neue substituierte Pyrimidylamidoxime der allgemeinen Formel (I)

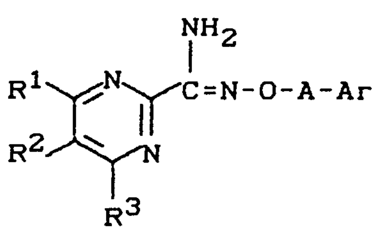

(I)

in welcher

$R^1$, $R^2$, $R^3$, A und Ar die in der Beschreibung gegebenen Bedeutungen haben, neue Zwischenprodukte zu ihrer Herstellung und ihre Verwendung zur Schädlingsbekämpfung.

Die neuen Verbindungen der Formel (I) können nach Analogieverfahren hergestellt werden, z.B. durch Umsetzung geeigneter Pyrimidylamidoximen mit geeigneten Alkylierungsmitteln. Die ebenfalls neuen Ausgangsverbindungen, die Pyrimidylamidoxime der Formel (II), in der -A-Ar für Wasserstoff steht, können aus geeigneten Cyanopyrimidinen mit Hydroxylamin oder dessen Säureaddukten hergestellt werden.

EP 0 490 188 A1

Die Erfindung betrifft neue substituierte Pyrimidylamidoxime, ein Verfahren und neue Zwischenprodukte zu ihrer Herstellung und ihre Verwendung als Schädlingsbekämpfungsmittel, insbesondere als Fungizide.

Pyrimidin-2-amidoxim ist bereits aus der Literatur bekannt (vgl. Ann. Chim. (Paris) 5 (1960), 351-379 - zitiert in Chem. Abstracts 56 (1962), 5961-5962). Über die biologischen Eigenschaften dieser Verbindung ist jedoch bisher nichts bekanntgeworden.

Bestimmte fungizid wirksame Oxime, wie z.B. α-(4-Methylphenylsulfonyl)-0-(4-methyl-phenylsulfonylaminocarbonyl)-2,6-dichlor-benzaldoxim und α-(4-Methyl-phenylsulfonyl)-0-(4-methyl-phenylaminocarbonyl)-2,6-dichlorbenzaldoxim (vgl. EP-A 205 076), α-(4-Chlor-phenylsulfonyl)-0-cyclohexylaminocarbonyl-2,6-dichlor-benzaldoxim (vgl. EP-A 281 901), α-(4-Chlor-phenylsulfonyl)-0-ethoxycarbonyl-pyridin-3-aldoxim und α-(4-Methyl-phenylsulfonyl)-0-ethoxycarbonyl-6-methyl-pyridin-2-aldoxim (vgl. EP-A 236 897) sowie α-(4-Methyl-phenylsulfonyl)-6-methyl-pyridin-2-aldoxim, α-(4-Chlor-phenylsulfonyl)-pyridin-4-aldoxim und α-Phenylsulfonyl-6-methyl-pyridin-2-aldoxim (vgl. EP-A 236 919), sind bereits bekannt. Die Wirkung dieser Verbindungen ist jedoch bei niedrigen Aufwandmengen nicht ganz zufriedenstellend.

Es wurden nun neue substituierte Pyrimidylamidoxime der allgemeinen Formel (I)

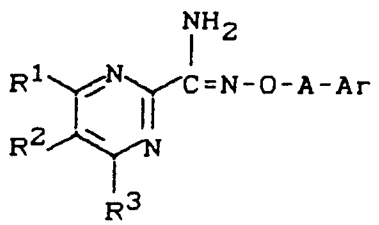

(I)

in welcher

| | |
|---|---|
| $R^1$, $R^2$ und $R^3$ | gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, wobei zwei dieser Reste $R^1$ und $R^2$ oder $R^2$ und $R^3$ auch zusammen für Alkandiyl stehen können, |
| A | für Alkandiyl steht und |
| Ar | für jeweils gegebenenfalls substituiertes Aryl oder Heteroaryl steht, |

gefunden.

Weiter wurde gefunden, daß man die neuen Verbindungen der Formel (I) erhält, wenn man Pyrimidylamidoxime der allgemeinen Formel (II)

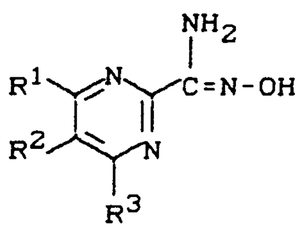

(II)

in welcher

| | |
|---|---|
| $R^1$, $R^2$ und $R^3$ | gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, wobei zwei dieser Reste $R^1$ und $R^2$ oder $R^2$ und $R^3$ auch zusammen für Alkandiyl stehen können, |

mit Alkylierungsmitteln der allgemeinen Formel (III)

X-A-Ar (III)

in welcher

| | |
|---|---|
| A | für Alkandiyl steht, |
| Ar | für jeweils gegebenenfalls substituiertes Aryl oder Heteroaryl steht und |

X für Halogen steht,

gegebenenfalls in Gegenwart eines Säureakzeptors und gegebenenfalls in Gegenwart eines Verdünnungsmittels umsetzt.

Die neuen substituierten Pyrimidylamidoxime der allgemeinen Formel (I) zeigen starke Wirkung als Schädlingsbekämpfungsmittel, insbesondere als Fungizide, daneben in gewissem Umfang auch als Insektizide.

Überraschenderweise zeigen die neuen Verbindungen der Formel (I) wesentlich stärkere fungizide Wirkung als die bekannten Verbindungen α-(4-Methyl-phenylsulfonyl)-0-(4-methyl-phenylsulfonylaminocarbonyl)-2,6-dichlor-benzaldoxim und α-(4-Methyl-phenylsulfonyl)-0-(4-methyl-phenylaminocarbonyl)-2,6-dichlor-benzaldoxim (vgl. EP-A 205 076), α-(4-Chlor-phenylsulfonyl)-0-cyclohexylaminocarbonyl-2,6-dichlor-benzaldoxim (vgl. EP-A 281 901), α-(4-Chlor-phenylsulfonyl)-0-ethoxycarbonyl-pyridin-3-aldoxim und α-(4-Methyl-phenylsulfonyl)-0-ethoxycarbonyl-6-methyl-pyridin-2-aldoxim (vgl. EP-A 236 897) sowie α-(4-Methyl-phenylsulfonyl-6-methyl-pyridin-2-aldoxim und α-Phenylsulfonyl-6-methyl-pyridin-2-aldoxim (vgl. EP-A 236 919).

Bevorzugte Substituenten werden im folgenden erläutert: Alkyl steht in den allgemeinen Formeln einzeln oder in zusammengesetzten Resten, wie z.B. Alkoxy, Alkylthio, Alkylamino, jeweils für geradkettiges oder verzweigtes Alkyl, vorzugsweise mit 1 bis 6, insbesondere mit 1 bis 4 Kohlenstoffatomen. Beispielhaft seien genannt: Methyl, Ethyl, n- und iso-Propyl, n-, iso-, sec- und tert-Butyl.

Halogenalkyl steht in den allgemeinen Formeln einzeln oder in zusammengesetzten Resten, wie z.B. Halogenalkoxy, jeweils für geradkettiges oder verzweigtes Halogenalkyl, vorzugsweise mit 1 bis 6 Kohlenstoffatomen und 1 bis 13 Halogenatomen, vorzugsweise Fluor- oder Chloratomen, insbesondere mit 1 bis 4 Kohlenstoffatomen und 1 bis 9 Halogenatomen. Beispielhaft seien genannt: Fluormethyl, Chlormethyl, Difluormethyl, Dichlormethyl, Trifluormethyl, Trichlormethyl, Chlorfluormethyl, Chlordifluormethyl, Fluordichlormethyl, Fluorethyl, Difluorethyl, Trifluorethyl, Tetrafluorethyl, Chlorethyl, Dichlorethyl, Trichlorethyl, Chlorfluorethyl, Chlordifluorethyl, Chlortrifluorethyl, Fluorpropyl, Chlorpropyl, Fluorbutyl und Chlorbutyl.

Alkandiyl steht in den allgemeinen Formeln jeweils für geradkettiges oder verzweigtes Alkandiyl, vorzugsweise mit 1 bis 5, insbesondere mit 1 bis 4 Kohlenstoffatomen. Beispielhaft seien genannt: Methan-1,1-diyl (Methylen), Ethan-1,1-diyl (Ethyliden), Ethan-1,2-diyl (Dimethylen), Propan-1,1-diyl (Propyliden), Propan-1,2-diyl, Propan1,3-diyl und Butan-1,4-diyl.

Halogen steht in den allgemeinen Formeln für Fluor, Chlor, Brom oder Iod, vorzugsweise für Fluor, Chlor oder Brom, insbesondere für Fluor oder Chlor.

Aryl steht in den allgemeinen Formeln vorzugsweise für Phenyl oder Naphthyl, insbesondere für Phenyl.

Heteroaryl steht in den allgemeinen Formeln vorzugsweise für fünfgliedrige oder sechsgliedrige, gegebenenfalls benzanellierte aromatische Heterocyclen mit bis zu 3 Stickstoffatomen und/oder gegebenenfalls einem Sauerstoff- oder Schwefelatom, insbesondere für Furyl, Thienyl, Pyrrolyl, Pyrazolyl, Imidazolyl, Triazolyl, Oxazolyl, Benzoxazolyl, Isoxazolyl, Thiazolyl, Benzthiazolyl, Oxadiazolyl, Thiadiazolyl, Pyridyl, Pyrimidyl und Triazinyl.

Die möglichen Substituenten für Aryl und Heteroaryl sind vorzugsweise ausgewählt aus der Reihe Halogen, Cyano, Carboxy, Nitro, Phenyl, jeweils geradkettiges oder verzweigtes Alkyl, Alkoxy, Alkylthio oder Alkoxycarbonyl vorzugsweise mit jeweils bis zu 4 Kohlenstoffatomen, Alkandiyl vorzugsweise mit 3 oder 4 Kohlenstoffatomen, Alkylendioxy vorzugsweise mit 1 oder 2 Kohlenstoffatomen, jeweils geradkettiges oder verzweigtes Halogenalkyl, Halogenalkyloxy oder Halogenalkylthio vorzugsweise mit jeweils bis zu 4 Kohlenstoffatomen und vorzugsweise bis zu 9 Halogenatomen, Halogenalkylendioxy vorzugsweise mit 1 oder 2 Kohlenstoffatomen und vorzugsweise bis zu 4 Halogenatomen, oder Phenoxy, welches gegebenenfalls die vorausgehend für Aryl und Heteroaryl genannten Substituenten enthält. Insbesondere sind die möglichen Substituenten für Aryl und Heteroaryl ausgewählt aus der Reihe Fluor, Chlor, Brom, Cyano, Methyl, Ethyl, Methoxy, Ethoxy, Methylthio, Ethylthio, Methoxycarbonyl, Ethoxycarbonyl, Trifluormethyl, Trifluormethoxy, Trifluormethylthio, Methylendioxy, Ethylendioxy, Difluormethylendioxy, Trifluorethylendioxy, Tetrafluorethylendioxy, Chlortrifluorethylendioxy, oder Phenoxy, welches gegebenenfalls die vorausgehend für Aryl und Heteroaryl genannten Substituenten enthält.

Die erfindungsgemäßen substituierten Pyrimidylamidoxime sind durch die Formel (I) allgemein definiert. Bevorzugt sind diejenigen Verbindungen der Formel (I), in welcher

$R^1$ für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_2$-Alkoxy-$C_1$-$C_2$-alkoxy, $C_1$-$C_4$-Alkylthio, $C_1$-$C_4$-Alkylamino oder Di-($C_1$-$C_2$-alkyl)-amino steht,

$R^2$ für Wasserstoff, Halogen, $C_1$-$C_4$-Alkyl, $C_1$-$C_4$-Halogenalkyl oder $C_1$-$C_4$-Alkoxy steht oder zusammen mit $R^1$ oder $R^3$ für Trimethylen oder Tetramethylen steht,

$R^3$ für Wasserstoff, Halogen, $C_1$-$C_4$-Alkyl, $C_1$-$C_4$-Halogenalkyl, $C_1$-$C_4$-Alkoxy oder $C_1$-$C_4$-Halogenalkoxy steht,

A für $C_1$-$C_5$-Alkandiyl steht und

Ar für jeweils gegebenenfalls substituiertes Phenyl oder Naphthyl oder für gegebenenfalls substituiertes und/oder gegebenenfalls benzanelliertes fünfgliedriges oder sechsgliedriges Heteroaryl mit bis zu 3 Stickstoffatomen und/oder gegebenenfalls einem Sauerstoff- oder Schwefelatom steht, wobei die möglichen Substituenten vorzugsweise ausgewählt sind aus der Reihe Halogen, Cyano, Carboxy, Nitro, Phenyl, jeweils geradkettiges oder verzweigtes Alkyl, Alkoxy, Alkylthio oder Alkoxycarbonyl mit jeweils bis zu 4 Kohlenstoffatomen, Alkandiyl mit 3 oder 4 Kohlenstoffatomen, Alkylendioxy mit 1 oder 2 Kohlenstoffatomen, jeweils geradkettiges oder verzweigtes Halogenalkyl, Halogenalkyloxy oder Halogenalkylthio mit jeweils bis zu 4 Kohlenstoffatomen und bis zu 9 Halogenatomen, Halogenalkylendioxy mit 1 oder 2 Kohlenstoffatomen und bis zu 4 Halogenatomen, oder Phenoxy, welches gegebenenfalls die vorausgehend für Aryl und Heteroaryl genannten Substituenten enthält.

Besonders bevorzugt sind diejenigen Verbindungen der Formel (I), in welcher

$R^1$ für Wasserstoff, Fluor, Chlor, Brom, Methyl, Ethyl, Trifluormethyl, Chlormethyl, Methoxymethyl, Methoxy, Ethoxy, Difluormethoxy, 2,2,2-Trifluorethoxy, 2-Methoxy-ethoxy, Methylthio, Methylamino, Ethylamino oder Dimethylamino steht,

$R^2$ für Wasserstoff, Fluor, Chlor, Brom, Methyl, Trifluormethyl oder Methoxy steht,

$R^3$ für Wasserstoff, Fluor, Chlor, Brom, Methyl, Ethyl, Propyl, Trifluormethyl, Chlormethyl, Methoxy, Ethoxy oder Difluormethoxy steht,

A für Methan-1,1-diyl (Methylen), Ethan-1,1-diyl (Ethyliden) oder Ethan-1,2-diyl (Dimethylen) steht, und

Ar für jeweils gegebenenfalls substituiertes Phenyl, Naphthyl, Furyl, Thienyl, Pyrrolyl, Pyrazolyl, Imidazolyl, Triazolyl, Oxazolyl, Benzoxazolyl, Isoxazolyl, Benzthiazolyl, Thiazolyl, Oxadiazolyl, Thiadiazolyl, Pyridyl, Pyrimidyl oder Triazinyl steht, wobei die möglichen Substituenten insbesondere ausgewählt sind aus der Reihe Fluor, Chlor, Brom, Cyano, Phenyl , Methyl, Ethyl, Methoxy, Ethoxy, Methylthio, Ethylthio, Methoxycarbonyl, Ethoxycarbonyl, Trifluormethyl, Trifluormethoxy, Trifluormethylthio, Methylendioxy, Ethylendioxy, Difluormethylendioxy, Trifluormethylendioxy, Tetrafluorethylendioxy, Chlortrifluorethylendioxy, oder Phenoxy, welches gegebenenfalls die vorausgehend für die Aryl- und Heteroarylreste genannten Substituenten enthält.

Die Aryl und/oder Heteroarylreste können bevorzugt gleich oder verschieden, ein- bis fünffach, insbesondere ein- bis vierfach und ganz besonders ein- bis dreifach substituiert sein.

Verwendet man beispielsweise 4,6-Dimethyl-pyrimidin-2-carbamidoxim und 3,4-Dichlor-benzylchlorid als Ausgangsstoffe, so kann der Reaktionsverlauf beim erfindungsgemäßen Herstellungsverfahren durch das folgende Formelschema wiedergegeben werden:

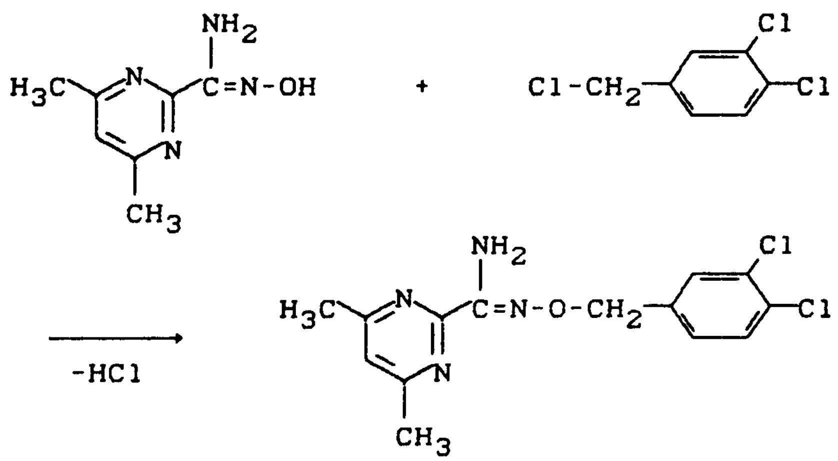


Die beim erfindungsgemäßen Verfahren zur Herstellung von Verbindungen der Formel (I) als Ausgangsstoffe zu verwendenden Pyrimidylamidoxime sind durch die Formel (II) allgemein definiert.

In Formel (II) haben $R^1$, $R^2$ und $R^3$ vorzugsweise bzw. insbesondere diejenigen Bedeutungen, die

bereits oben im Zusammenhang mit der Beschreibung der erfindungsgemäßen Verbindungen der Formel (I) vorzugsweise bzw. als insbesondere bevorzugt für $R^1$, $R^2$ und $R^3$ angegeben wurden.

Als Beispiele für die Ausgangsstoffe der Formel (II) seien genannt:

Pyrimidin-2-carbamidoxim, 4-Methyl-, 5-Methyl-, 4,5-Dimethyl-, 4,6-Dimethyl-, 4,5,6-Trimethyl-, 4-Methoxy-, 5-Methoxy-, 4-Methoxy-6-methyl-, 4-Methoxy-5-methyl-, 5-Methoxy-4-methyl-, 4,6-Dimethoxy-, 4-Methoxy-6-ethyl-, 4-Ethoxy-6-methyl-, 4-Methoxy-6-trifluormethyl-, 4,6-Diethoxy- und Bis-4,6-difluormethoxy-pyrimidin-Z-carbamidoxim.

Die Ausgangsstoffe der Formel (II) sind mit Ausnahme von Pyrimidin-2-carbamidoxim (Pyrimidin-2-amidoxim, - (II), $R^1 = R^2 = R^3 = H$ - vgl. Ann. Chim. (Paris) 5 (1960), 351-379) neu und Teil der vorliegenden Erfindung.

Man erhält die neuen Pyrimidylamidoxime der Formel (II), wenn man Cyanopyrimidine der allgemeinen Formel (IV)

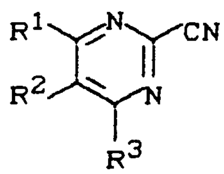

(IV)

in welcher

$R^1$, $R^2$ und $R^3$ die oben angegebene Bedeutung haben,

mit Hydroxylamin oder dessen Säureaddukten, wie z.B. Hydroxylammoniumchlorid, gegebenenfalls in Gegenwart eines Säureakzeptors, wie z.B. Natriumcarbonat, und gegebenenfalls in Gegenwart von Verdünnungsmitteln, wie z.B. Ethanol und Wasser, bei Temperaturen zwischen 20° C und 100° C umsetzt.

In Formel (IV) haben $R^1$, $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$ und $R^3$ angegeben wurden.

Als Beispiele für die Verbindungen der Formel (IV) seien genannt:

4,5-Dimethyl-, 4,6-Dimethyl-, 4,5,6-Trimethyl-, 4-Methoxy-, 5-Methoxy-, 4-Methoxy-6-methyl-, 4-Methoxy-5-methyl-, 5-Methoxy-4-methyl-, 4,6-Dimethoxy-, 4-Methoxy-6-ethyl-, 4-Ethoxy-6-methyl-, 4-Methoxy-6-trifluormethyl-, 4,6-Diethoxy- und Bis-4,6-difluormethoxy-pyrimidin-2-carbonitril.

Die Cyanopyrimidine der Formel (IV) sind bekannt und/oder können nach an sich bekannten Verfahren hergestellt werden (vgl. Beilstein E III/IV, Band 25, 783-791; Monatshefte Chemie 87 (1956), 526-546; Chem. Pharm. Bull. 6 (1958), 633-638).

Die beim erfindungsgemäßen Verfahren zur Herstellung von Verbindungen der Formel (I) weiter als Ausgangsstoffe zu verwendenden Alkylierungsmittel sind durch die Formel (III) allgemein definiert.

In Formel (III) haben A und Ar vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A und Ar angegeben wurden; X steht für Fluor, Chlor, Brom oder Iod, vorzugsweise für Chlor oder Brom.

Als Beispiele für die Ausgangsstoffe der Formel (III) seien genannt:

Benzylchlorid, 1-Phenyl- und 2-Phenyl-ethylchlorid, 2-Fluor-, 3-Fluor-, 4-Fluor-, 2,4-Difluor-, 2,6-Difluor- und 3,4-Difluor-benzylchlorid, 2-Chlor-, 3-Chlor-, 4-Chlor-, 2,4-Dichlor-, 2,6-Dichlor- und 3,4-Dichlor-benzylchlorid, 2-Brom-, 3-Brom- und 4-Brom-benzylchlorid, 4-Cyano-benzylchlorid, 2-Methyl-, 3-Methyl-, 4-Methyl-, 2,3-Dimethyl-, 2,4-Dimethyl-, 2,6-Dimethyl-, 2,5-Dimethyl-, 3,4-Dimethyl- und 2,4,6-Trimethyl-benzylchlorid, 2-Methoxy-, 3-Methoxy-, 4-Methoxy- und 3,4-Dimethoxy-benzylchlorid, 3-Methoxycarbonyl- und 4-Methoxycarbonyl-benzylchlorid, 2-Trifluormethyl-, 3-Trifluormethyl- und 4-Trifluormethyl-benzylchlorid, 4-Trifluormethoxy- und 4-Trifluormethylthio-benzylchlorid, 3,4-Methylendioxy-benzylchlorid, 4-Phenoxy- und 3-Phenoxy-benzylchlorid, 1-Chlormethyl- und 2-Chlormethyl-naphthalin sowie die entsprechenden Bromalkylverbindungen.

Die Ausgangsstoffe der Formel (III) sind bekannte Synthesechemikalien.

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und

Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren alle üblicherweise für derartige Umsetzungen verwendbaren Säurebindemittel eingesetzt werden. Vorzugsweise infrage kommen Alkalimetall- und Erdalkalimetallhydride, wie Lithium-, Natrium-, Kalium- und Calcium-hydrid, Alkalimetall- und Erdalkalimetall-hydroxide, wie Lithium-, Natrium-, Kalium- und Calcium-hydroxid, Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden. Im allgemeinen arbeitet man bei Temperaturen zwischen 0 ° C und 150 ° C, vorzugsweise bei Temperaturen zwischen 20 ° C und 120 ° C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die erfindungsgemäßen Wirkstoffe weisen eine starke biologische Wirkung auf und können zur Bekämpfung von Schädlingen praktisch eingesetzt werden. Die Wirkstoffe sind für den Gebrauch vorzugsweise in Pflanzenschutzmitteln einsetzbar, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium); Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;

Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Verbindungen der Formel (I) zeigen insbesondere starke protektive Wirkung gegen Venturia-Arten, wie z.B. gegen Venturia inaequalis an Äpfeln, gegen Cochliobolus-Arten, wie z.B. Cochliobolus sativus an Gerste, und gegen Pyrenophora-Arten, wie z.B. gegen Pyrenophora teres an Gerste. Auch gegen Pyricularia oryzae an Reis ist eine starke Wirkung zu beobachten.

In gewissem Umfang können auch weitere pilzliche Pflanzenschädlinge, wie z.B. Erysiphe graminis, Fusarium nivale und Septoria nodorum an Getreide, aber auch tierische Schädlinge, wie Insekten und Nematoden mit den Verbindungen der Formel (I) bekämpft werden.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder Schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und Synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werdend Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1:

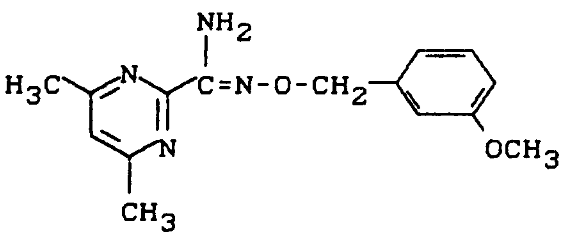

Eine Mischung aus 2,5 g (15 mmol) 4,6-Dimethyl-pyrimidin-2-carbamidoxim, 2,82 g (15 mmol) 3-Methoxy-benzylchlorid, 2,07 g (15 mmol) Kaliumcarbonat und 50 ml Acetonitril wird unter Rühren 14 Stunden zu leichtem Rückfluß erhitzt. Anschließend wird auf Raumtemperatur abgekühlt, filtriert und das Filtrat im Wasserstrahlvakuum eingeengt. Der Rückstand wird durch Verrühren mit Petrolether zur Kristallisation gebracht und das kristalline Produkt wird durch Absaugen isoliert.

Man erhält 3,7 g (86% der Theorie) 0-(3-Methoxy-benzyl)-4,6-dimethyl-pyrimidin-2-carbamidoxim vom Schmelzpunkt 83° C.

Analog Beispiel 1 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

<u>Tabelle 1:</u> Beispiele für die Verbindungen der Formel (I)

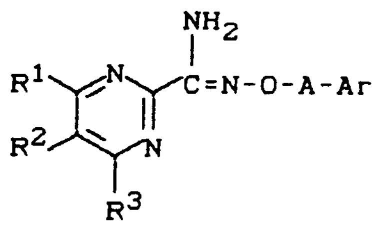

(I)

| Bsp.-Nr. | $R^1$ | $R^2$ | $R^3$ | A | Ar | Physik. Daten |
|---|---|---|---|---|---|---|
| 2 | H | $OCH_3$ | H | $CH_2$ | 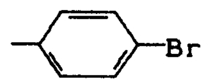 | Fp: 138° C |
| 3 | H | $OCH_3$ | H | $CH_2$ | 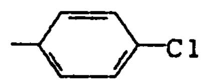 | Fp: 121° C |
| 4 | $CH_3$ | H | $OCH_3$ | $CH_2$ | 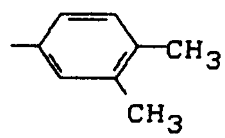 | (Öl) |
| 5 | $CH_3$ | $CH_3$ | $CH_3$ | $CH_2$ | 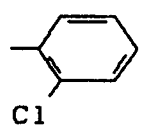 | Fp: 152° C |
| 6 | $CH_3$ | $CH_3$ | $CH_3$ | $CH(CH_3)$ | 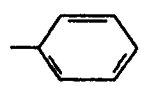 | Fp: 115° C |

| Bsp.-Nr. | $R^1$ | $R^2$ | $R^3$ | A | Ar | Physik. Daten |
|---|---|---|---|---|---|---|
| 7 | $CH_3$ | $CH_3$ | $CH_3$ | $CH_2$ | 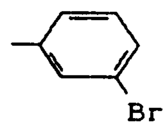 | Fp: 93° C |
| 8 | $CH_3$ | $CH_3$ | $CH_3$ | $CH_2$ | 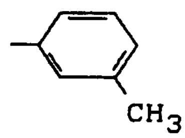 | Fp: 94° C |
| 9 | $CH_3$ | $CH_3$ | $CH_3$ | $CH_2$ | 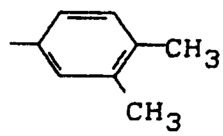 | Fp: 103° C |
| 10 | $CH_3$ | $CH_3$ | $CH_3$ | $CH_2$ | 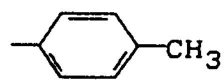 | Fp: 143° C |
| 11 | $CH_3$ | $CH_3$ | $CH_3$ | $CH_2$ | 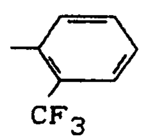 | Fp: 111° C |
| 12 | $CH_3$ | H | $OCH_3$ | $CH_2$ | 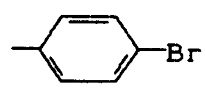 | Fp: 114° C |
| 13 | $OCH_3$ | H | $OCH_3$ | $CH_2$ | 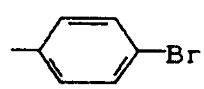 | (Öl) |
| 14 | $CH_3$ | $CH_3$ | $CH_3$ | $CH_2CH_2$ | 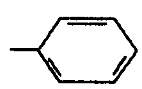 | Fp: 105° C |
| 15 | $CH_3$ | H | $CH_3$ | $CH_2$ | 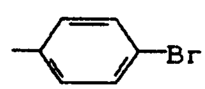 | Fp: 112° C |
| 16 | $CH_3$ | H | $CH_3$ | $CH_2$ | 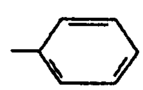 | δ = 5,30*) |

| Bsp.-Nr. | $R^1$ | $R^2$ | $R^3$ | A | Ar | Physik. Daten |
|---|---|---|---|---|---|---|
| 17 | $CH_3$ | H | $CH_3$ | $CH_2$ | 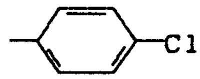 | (Öl) |
| 18 | $CH_3$ | H | $CH_3$ | $CH_2$ | 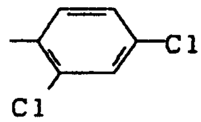 | Fp: 121° C |
| 19 | $CH_3$ | H | $CH_3$ | $CH_2$ | 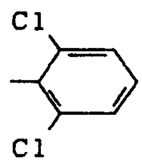 | (Öl) |
| 20 | $CH_3$ | H | $CH_3$ | $CH_2$ | 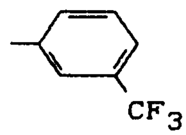 | $\delta$ = 5,35*) |
| 21 | $CH_3$ | H | $CH_3$ | $CH_2$ | 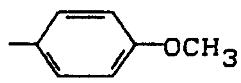 | $\delta$ = 5,29*) |
| 22 | $CH_3$ | H | $CH_3$ | $CH_2$ | 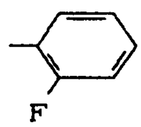 | Fp: 122° C |
| 23 | $CH_3$ | H | $CH_3$ | $CH_2$ | 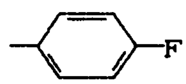 | Fp: 59° C |
| 24 | $CH_3$ | H | $CH_3$ | $CH_2$ | 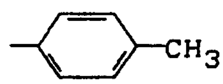 | $\delta$ = 5,26*) |
| 25 | $CH_3$ | H | $CH_3$ | $CH_2$ | 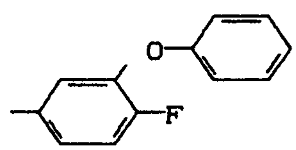 | $\delta$ = 5,21*) |
| 26 | $CH_3$ | H | $CH_3$ | $CH_2$ | 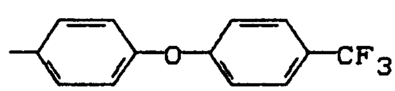 | $\delta$ = 5,30*) |

| Bsp.-Nr. | $R^1$ | $R^2$ | $R^3$ | A | Ar | Physik. Daten |
|---|---|---|---|---|---|---|
| 27 | $CH_3$ | H | $CH_3$ | $CH_2$ | 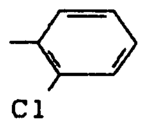 | Fp: 101° C |
| 28 | $CH_3$ | H | $CH_3$ | $CH_2$ | 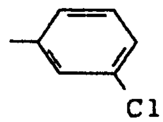 | Fp: 77° C |
| 29 | $CH_3$ | H | $CH_3$ | $CH_2$ | 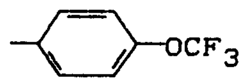 | δ = 5,29*) |
| 30 | $CH_3$ | H | $CH_3$ | $CH_2$ | 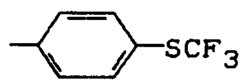 | δ = 5,34*) |
| 31 | $CH_3$ | H | $CH_3$ | $CH_2$ | 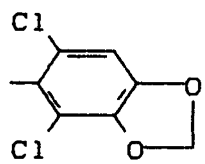 | Fp: 127° C |
| 32 | $CH_3$ | H | $OCH_3$ | $CH_2$ | 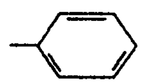 | δ = 5,28*) |
| 33 | $CH_3$ | H | $OCH_3$ | $CH_2$ | 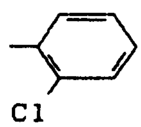 | δ = 5,40*) |
| 34 | $CH_3$ | H | $OCH_3$ | $CH_2$ | 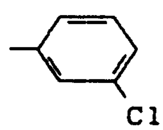 | δ = 5,24*) |
| 35 | $CH_3$ | H | $CH_3$ | $CH_2$ | 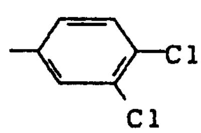 | Fp: 81° C |

| Bsp.-Nr. | $R^1$ | $R^2$ | $R^3$ | A | Ar | Physik. Daten |
|---|---|---|---|---|---|---|
| 36 | $CH_3$ | H | $CH_3$ | $CH_2$ | 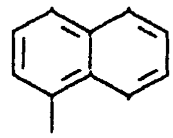 | Fp: 85° C |
| 37 | $CH_3$ | H | $OCH_3$ | $CH_2$ | 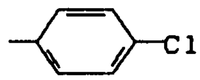 | Fp: 87° C |
| 38 | $CH_3$ | H | $CH_3$ | $CH_2$ | 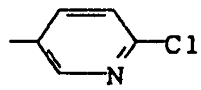 | (Öl) |
| 39 | $CH_3$ | H | $CH_3$ | $CH_2$ | 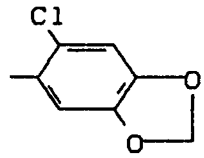 | (Öl) |
| 40 | $CH_3$ | H | H | $CH_2$ | 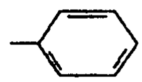 | (Öl) |
| 41 | $CH_3$ | H | H | $CH_2$ | 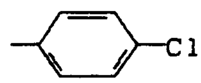 | (Öl) |
| 42 | $CH_3$ | H | H | $CH_2$ | 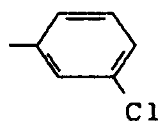 | Fp: 110° C |
| 43 | $CH_3$ | H | H | $CH_2$ | 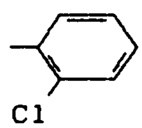 | Fp: 135° C |
| 44 | $CH_3$ | H | $CH_3$ | $CH_2$ | 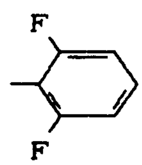 | Fp: 128° C |
| 45 | $CH_3$ | H | $CH_3$ | $CH_2CH_2$ | 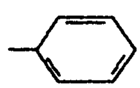 | (Öl) |

| Bsp.-Nr. | $R^1$ | $R^2$ | $R^3$ | A | Ar | Physik. Daten |
|---|---|---|---|---|---|---|
| 46 | $OCH_3$ | H | $C_3H_7$ | $CH_2$ | 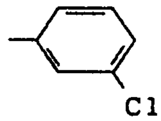 | δ = 5,23*) |
| 47 | $CH_3$ | H | $CH_3$ | $CH_2$ | 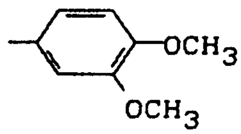 | (Öl) |
| 48 | $CH_3$ | H | $CH_3$ | $CH_2$ | 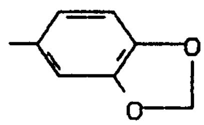 | (Öl) |
| 49 | $CH_3$ | H | H | $CH_2$ | 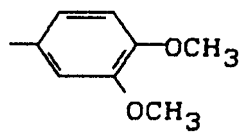 | (Öl) |
| 50 | $CH_3$ | H | H | $CH_2$ | 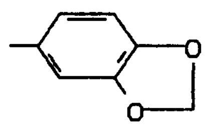 | (Öl) |
| 51 | $CH_3$ | H | $CH_3$ | $CH_2$ | 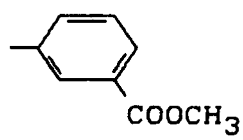 | (Öl) |
| 52 | $CH_3$ | H | $OCH_3$ | $CH_2$ | 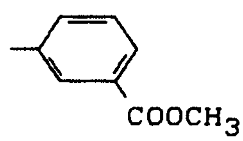 | (Öl) |
| 53 | $OCH_3$ | H | H | $CH_2$ | 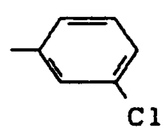 | (Öl) |
| 54 | $OCH_3$ | $-(CH_2)_3-$ | | $CH_2$ | 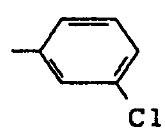 | δ = 5,25*) |

| Bsp.-Nr. | $R^1$ | $R^2$ | $R^3$ | A | Ar | Physik. Daten |
|---|---|---|---|---|---|---|
| 55 | $CH_3$ | H | $CH_3$ | $CH_2$ | 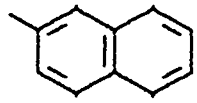 | δ = 5,46*) |
| 56 | $CH_3$ | H | $OCH_3$ | $CH_2$ | 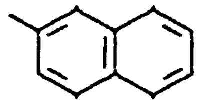 | δ = 5,44*) |
| 57 | $CH_3$ | $CH_3$ | $CH_3$ | $CH_2$ | 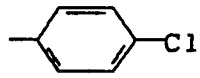 | Fp: 101° C |
| 58 | $CH_3$ | $CH_3$ | $CH_3$ | $CH_2$ | 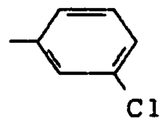 | Fp: 89° C |
| 59 | $CH_3$ | $CH_3$ | $CH_3$ | $CH_2$ | 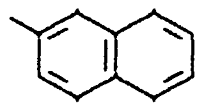 | Fp: 95° C |
| 60 | $OCH_3$ | H | $OCH_3$ | $CH_2$ | 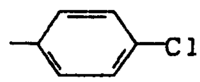 | (Öl) |
| 61 | $OCH_3$ | H | $OCH_3$ | $CH_2$ | 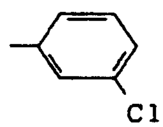 | (Öl) |
| 62 | $OCH_3$ | H | $OCH_3$ | $CH_2$ | 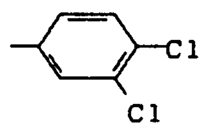 | (Öl) |
| 63 | $OCH_3$ | H | $OCH_3$ | $CH_2$ | 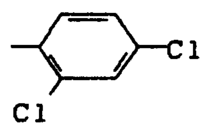 | (Öl) |
| 64 | $OCH_3$ | H | $OCH_3$ | $CH_2$ | 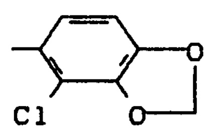 | (Öl) |

| Bsp.-Nr. | $R^1$ | $R^2$ | $R^3$ | A | Ar | Physik. Daten |
|---|---|---|---|---|---|---|
| 65 | $CH_3$ | H | $OCH_3$ | $CH_2$ | 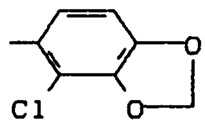 | (Öl) |
| 66 | $CH_3$ | H | $OCH_3$ | $CH_2$ | 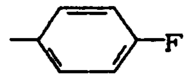 | (Öl) |
| 67 | $CH_3$ | H | $OCH_3$ | $CH_2$ | 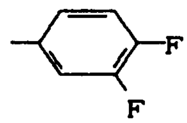 | (Öl) |
| 68 | $CH_3$ | H | $OCH_3$ | $CH_2$ | 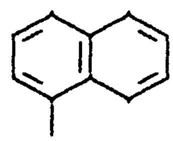 | Fp: 89° C |
| 69 | $CH_3$ | H | $OCH_3$ | $CH_2$ | 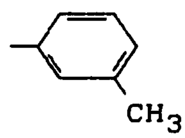 | $\delta$ = 5,25*) |
| 70 | $CH_3$ | H | $OCH_3$ | $CH_2$ | 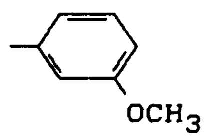 | (Öl) |
| 71 | $CH_3$ | H | $OCH_3$ | $CH_2$ | 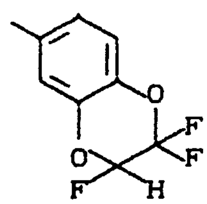 | (Öl) |
| 72 | $OC_2H_5$ | H | $OC_2H_5$ | $CH_2$ | 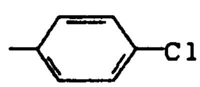 | (Öl) |
| 73 | $OC_2H_5$ | H | $OC_2H_5$ | $CH_2$ | 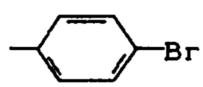 | (Öl) |

| Bsp.-Nr. | $R^1$ | $R^2$ | $R^3$ | A | Ar | Physik. Daten |
|---|---|---|---|---|---|---|
| 74 | $CH_3$ | H | H | $CH_2$ | 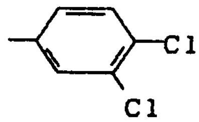 | (Öl) |
| 75 | $CH_3$ | H | H | $CH_2$ | 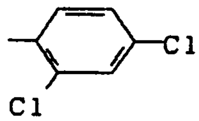 | (Öl) |
| 76 | $CH_3$ | H | H | $CH_2$ | 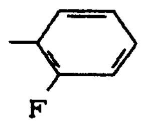 | (Öl) |
| 77 | $CH_3$ | H | $OCH_3$ | $CH_2$ | 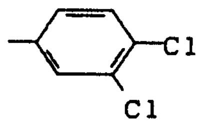 | $\delta$ = 5,20*) |
| 78 | $CH_3$ | H | $OCH_3$ | $CH_2$ | 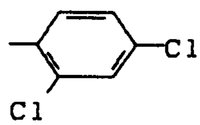 | Fp: 127° C |
| 79 | $CH_3$ | H | H | $CH_2$ | 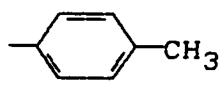 | (Öl) |
| 80 | $CH_3$ | H | H | $CH_2$ | 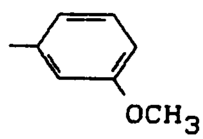 | (Öl) |
| 81 | $CH_3$ | H | H | $CH_2$ | 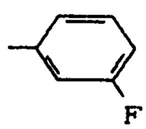 | (Öl) |
| 82 | $CH_3$ | H | H | $CH_2$ | 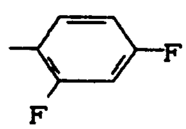 | (Öl) |

| Bsp.-Nr. | $R^1$ | $R^2$ | $R^3$ | A | Ar | Physik. Daten |
|---|---|---|---|---|---|---|
| 83 | $CH_3$ | H | $OCH_3$ | $CH_2$ | 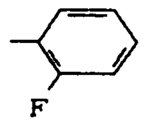 | Fp: 98° C |
| 84 | $CH_3$ | H | $OCH_3$ | $CH_2$ | 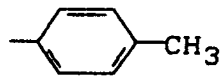 | δ = 5,16*) |
| 85 | $CH_3$ | H | H | $CH_2$ | 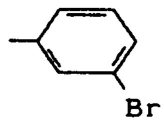 | (Öl) |
| 86 | $CH_3$ | H | H | $CH_2$ | 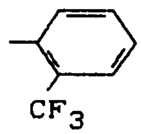 | (Öl) |
| 87 | $CH_3$ | H | $CH_3$ | $CH_2$ | 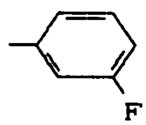 | Fp: 69° C |
| 88 | $CH_3$ | H | $CH_3$ | $CH_2$ | 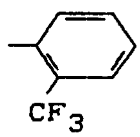 | Fp: 81° C |
| 89 | $CH_3$ | H | $OCH_3$ | $CH_2$ | 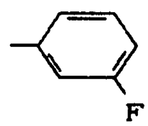 | δ = 5,27*) |
| 90 | $CH_3$ | H | $OCH_3$ | $CH_2$ | 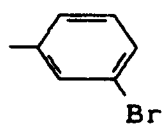 | δ = 5,23*) |
| 91 | $CH_3$ | H | $CH_3$ | $CH_2$ | 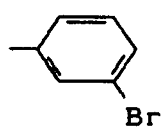 | (Öl) |

| Bsp.-Nr. | $R^1$ | $R^2$ | $R^3$ | A | Ar | Physik. Daten |
|---|---|---|---|---|---|---|
| 92 | $CH_3$ | H | $CH_3$ | $CH_2$ | 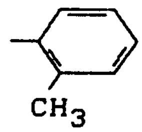 | Fp: 143° C |
| 93 | $CH_3$ | H | $CH_3$ | $CH_2$ | 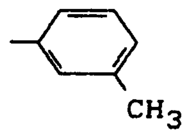 | (Öl) |
| 94 | $CH_3$ | H | $OCH_3$ | $CH_2$ | 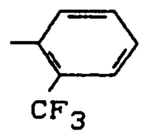 | (Öl) |
| 95 | $CH_3$ | H | $CH_3$ | $CH(CH_3)$ | 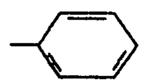 | (Öl) |
| 96 | $OCH_3$ | $-(CH_2)_3-$ | | $CH_2$ | 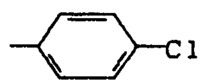 | (Öl) |
| 97 | H | $CH_3$ | H | $CH_2$ | 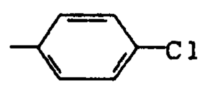 | (Öl) |
| 98 | $CH_3$ | H | $OCH_3$ | $CH_2$ | 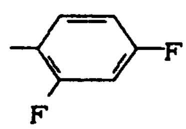 | (Öl) |
| 99 | $N(CH_3)_2$ | H | $OCH_3$ | $CH_2$ | 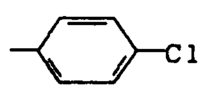 | (Öl) |
| 100 | $CH_3$ | H | $CH_3$ | $CH_2$ | 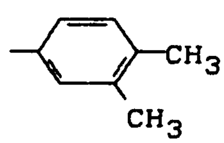 | Fp: 76° C |
| 101 | $CH_3$ | H | $CH_3$ | $CH_2$ | 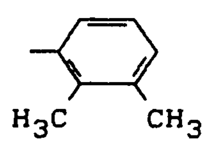 | (Öl) |

| Bsp.-Nr. | $R^1$ | $R^2$ | $R^3$ | A | Ar | Physik. Daten |
|---|---|---|---|---|---|---|
| 102 | $CH_3$ | H | $CH_3$ | $CH_2$ | 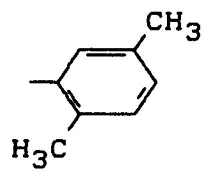 | Fp: 128° C |
| 103 | $CH_3$ | H | $CH_3$ | $CH_2$ | 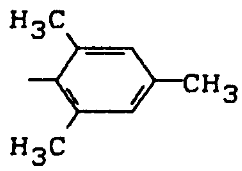 | (Öl) |
| 104 | $CH_3$ | H | $CH_3$ | $CH_2$ | 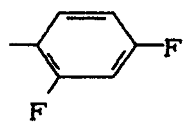 | (Öl) |
| 105 | $CH_3$ | H | $CH_3$ | $CH_2$ | 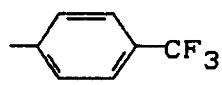 | Fp: 123° C |
| 106 | $CH_3$ | H | $CH_3$ | $CH_2$ | 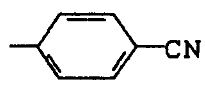 | (Öl) |
| 107 | $CH_3$ | H | $CH_3$ | $CH_2$ | 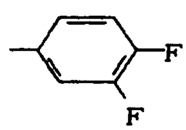 | (Öl) |
| 108 | $OCH_3$ | H | $C_3H_7$ | $CH_2$ | 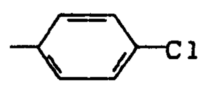 | (Öl) |
| 109 | $OCH_3$ | H | H | $CH_2$ | 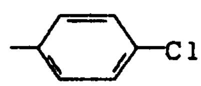 | (Öl) |
| 110 | $OCH_3$ | H | $CH_3$ | $CH_2$ | 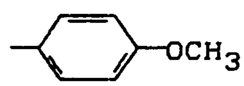 | Fp.: 96° C |
| 111 | $CH_3$ | $CH_3$ | $CH_3$ | $CH_2$ | 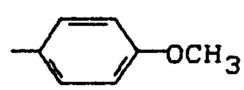 | Fp.:135° C |

| Bsp.-Nr. | $R^1$ | $R^2$ | $R^3$ | A | Ar | Physik. Daten |
|---|---|---|---|---|---|---|
| 112 | $CH_3$ | $CH_3$ | $CH_3$ | $CH_2$ | 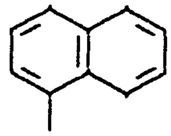 | Öl |
| 113 | $CH_3$ | $CH_3$ | $CH_3$ | $CH_2$ | 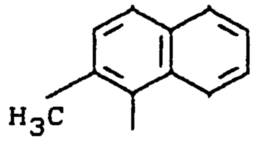 | Fp.: 139°C |
| 114 | $CH_3$ | H | $CH_3$ | $CH_2$ | 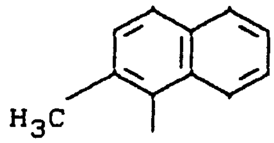 | Fp.: 151°C |
| 115 | $OCH_3$ | H | $CH_3$ | $CH_2$ | 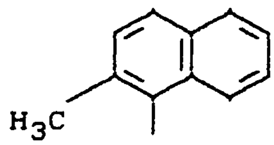 | Fp.: 96°C |
| 116 | $CH_3$ | H | $CH_3$ | $CH_2$ | 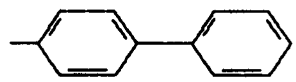 | (Öl) |
| 117 | $CH_3$ | $CH_3$ | $CH_3$ | $CH_2$ | 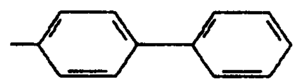 | Fp.: 121°C |
| 118 | $CH_3$ | $CH_3$ | $CH_3$ | $CH_2$ | $-C_5F_5$ | Fp.: 131°C |
| 119 | $CH_3$ | H | $CH_3$ | $CH_2$ | $-C_6F_5$ | Fp.: 101°C |
| 120 | $CH_3$ | H | $CH_3$ | $CH_2$ | 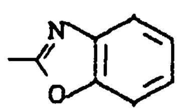 | Fp.: 132°C |

*) $^1H$-NMR: $\delta$-Werte (ppm) für A=$CH_2$ (in $CDCl_3$)

Ausgangsstoffe der Formel (II)

Beispiel (II-I):

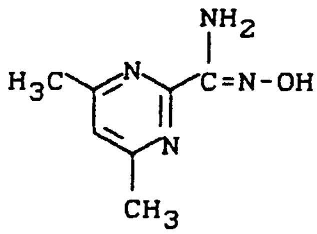

6,95 g (0,1 Mol) Hydroxylamin-hydrochlorid werden in 100 ml Ethanol gelöst. Dann wird eine Lösung von 10,6 g (0,1 Mol) Natriumcarbonat in 20 ml Wasser und anschließend 13,3 g (0,1 Mol) 4,6-Dimethyl-pyrimidin-2-carbonsäurenitril zugegeben. Die Lösung wird 14 Stunden unter Rückfluß gekocht, abgekühlt und eingeengt. Man nimmt den Rückstand in 200 ml Methylenchlorid auf, wäscht die Methylenchloridlösung mit wenig Wasser, trocknet sie über Natriumsulfat, filtriert und engt ein.

Man erhält 10,5 g (63% der Theorie) 4,6-Dimethyl-pyrimidin-2-carbamidoxim als kristallinen Rückstand vom Schmelzpunkt 209° C.

Analog Beispiel (II-1) können beispielsweise auch die in der nachstehenden Tabelle 2 aufgeführten Verbindungen der Formel (II) hergestellt werden.

Tabelle 2: Beispiele für die Verbindungen der Formel (II)

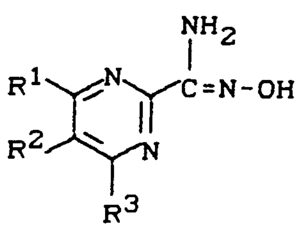

(II)

| Bsp.-Nr. | $R^1$ | $R^2$ | $R^3$ | Physikalische Daten |
|---|---|---|---|---|
| II-2 | $CH_3$ | H | H | Fp.: 212° C |
| II-3 | $OC_2H_5$ | H | $OC_2H_5$ | |
| II-4 | $CH_3$ | $CH_3$ | $CH_3$ | Fp.: 260° C |
| II-5 | $OCH_3$ | H | $C_3H_7$ | |
| II-6 | $OCH_3$ | H | H | |
| II-7 | $OCH_3$ | $-(CH_2)_3-$ | | |
| II-8 | H | $OCH_3$ | H | Fp.: 205° C |
| II-9 | $CH_3$ | H | $OCH_3$ | Fp.: 184° C |

Verwendungsbeispiele:

In den folgenden Verwendungsbeispielen werden die nachstehend aufgeführten Verbindungen als Vergleichssubstanzen eingesetzt:

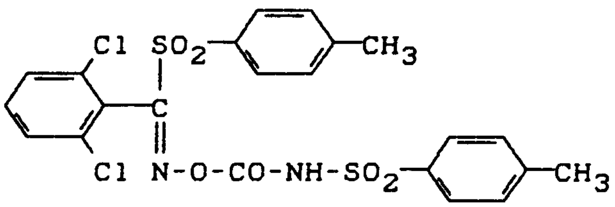

(A)

α-(4-Methyl-phenylsulfonyl)-0-(4-methyl-phenylsulfonylaminocarbonyl)-2,6-dichlor-benzaldoxim (bekannt aus EP-A 205 076, Bsp. 1);

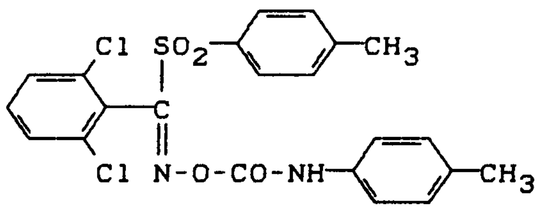

(B)

α-(4-Methyl-phenylsulfonyl)-0-(4-methyl-phenylaminocarbonyl)-2,6-dichlor-benzaldoxim (bekannt aus EP-A 205 076, Bsp. 16);

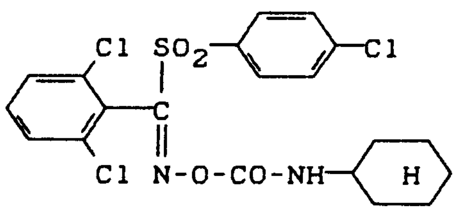

(C)

α-(4-Chlor-phenylsulfonyl)-0-cyclohexylaminocarbonyl-2,6-dichlor-benzaldoxim (bekannt aus EP-A 281 909, Bsp. 60);

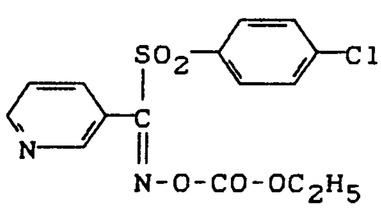

(D)

α-(4-Chlor-phenylsulfonyl)-0-ethoxycarbonyl-pyridin-3-aldoxim (bekannt aus EP-A 236 897, Bsp. 5);

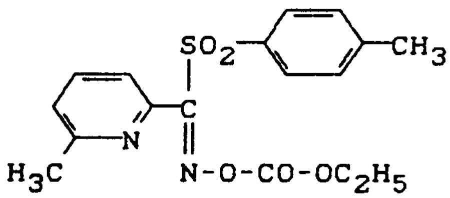

(E)

α-(4-Methyl-phenylsulfonyl)-0-ethoxycarbonyl-6-methylpyridin-2-aldoxim (bekannt aus EP-A 236 897, Bsp. 10);

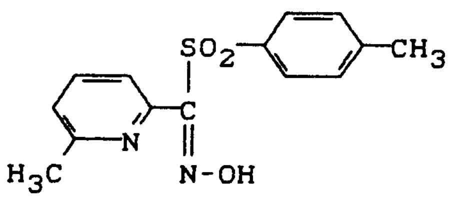

(F)

α-(4-Methyl-phenylsulfonyl)-6-methyl-pyridin-2-aldoxim (bekannt aus EP-A 236 919, Bsp. 10);

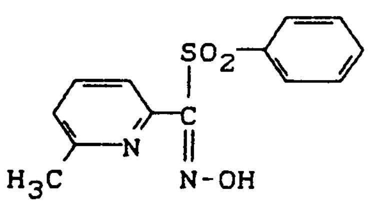

(G)

α-Phenylsulfonyl-6-methyl-pyridin-2-aldoxim (bekannt aus EP-A 236 919, Bsp. 11);

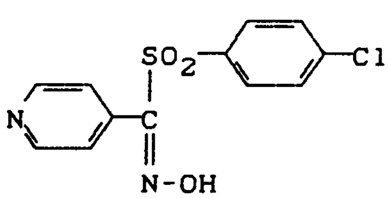

(H)

α-(4-Chlor-phenylsulfonyl)-pyridin-4-aldoxim (bekannt aus EP-A 236 919, Bsp. 3).

Beispiel A

Pyricularia-Test (Reis)/protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung

bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 ° C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 12, 22, 28, 29, 30, 32, 54, 57, 58, 59 und 69.

Beispiel B

Venturia-Test (Apfel) / protektiv

Lösungsmittel: 4,7 Gewichtsteile Aceton
Emulgator: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 ° C und 100 % relativer Luftfeuchtigkeit in einer Inkubationskabine.

Die Pflanzen werden dann im Gewächshaus bei 20° C und einer relativen Luftfeuchtigkeit von ca. 70% aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 12, 15, 18, 20, 21, 22, 23, 24, 25, 26, 27, 28, 29, 33, 34, 35, 36, 37, 42, 43, 46, 55, 69, 77, 83, 87, 90, 91 und 105.

Beispiel C

Cochliobolus sativus-Test (Gerste)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 ° C und 100 % rel. Luftfeuchtigkeit in einer Inkubationskabine.

Die Pflanzen werden in einem Gewächshaus bei einer Temperatur von ca. 20 ° C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 12, 16, 31, 35, 36, 44, 55, 68, 77, 83, 84, 88 und 89.

Beispiel D

Pyrenophora teres-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung

taufeucht. Nach Abtrocknen des Spritzbelages werden die Pflanzen mit einer Konidiensuspension von Pyrenophora teres besprüht. Die Pflanzen verbleiben 48 Stunden bei 20 ° C und 100 % relativer Luftfeuchtigkeit in einer Inkubationskabine.

Die Pflanzen werden in einem Gewächshaus bei einer Temperatur von ca. 20 ° C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 1, 12, 15, 18, 20, 21, 22, 23, 24, 27, 28, 32, 33, 34, 37, 56, 68, 77, 88, 90 und 92.

## Patentansprüche

**1.** Substituierte Pyrimidylamidoxime der allgemeinen Formel (I)

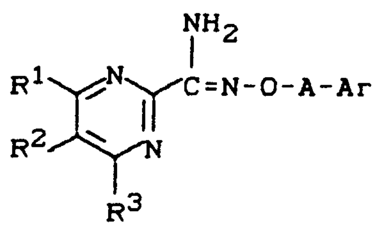

(I)

in welcher

- $R^1$, $R^2$ und $R^3$ gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, wobei zwei dieser Reste $R^1$ und $R^2$ oder $R^2$ und $R^3$ auch zusammen für Alkandiyl stehen können,
- A für Alkandiyl steht und
- Ar für jeweils gegebenenfalls substituiertes Aryl oder Heteroaryl steht.

**2.** Substituierte Pyrimidylamidoxime der Formel (I) gemäß Anspruch 1, in welcher

- $R^1$ für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_2$-Alkoxy-$C_1$-$C_2$-alkoxy, $C_1$-$C_4$-Alkylthio, $C_1$-$C_4$-Alkylamino oder Di-($C_1$-$C_2$-alkyl)-amino steht,
- $R^2$ für Wasserstoff, Halogen, $C_1$-$C_4$-Alkyl, $C_1$-$C_4$-Halogenalkyl oder $C_1$-$C_4$-Alkoxy steht oder zusammen mit R1 oder $R^3$ für Trimethylen oder Tetramethylen steht,
- $R^3$ für Wasserstoff, Halogen, $C_1$-$C_4$-Alkyl, $C_1$-$C_4$-Halogenalkyl, $C_1$-$C_4$-Alkoxy oder $C_1$-$C_4$-Halogenalkoxy steht,
- A für $C_1$-$C_5$-Alkandiyl steht und
- Ar für jeweils gegebenenfalls substituiertes Phenyl oder Naphthyl oder für gegebenenfalls substituiertes und/oder gegebenenfalls benzanelliertes fünfgliedriges oder sechsgliedriges Heteroaryl mit bis zu 3 Stickstoffatomen und/oder gegebenenfalls einem Sauerstoff- oder Schwefelatom steht, wobei die möglichen Substituenten vorzugsweise ausgewählt sind aus der Reihe Halogen, Cyano, Carboxy, Nitro, Phenyl, jeweils geradkettiges oder verzweigtes Alkyl, Alkoxy, Alkylthio oder Alkoxycarbonyl mit jeweils bis zu 4 Kohlenstoffatomen, Alkandiyl mit 3 oder 4 Kohlenstoffatomen, Alkylendioxy mit 1 oder 2 Kohlenstoffatomen, jeweils geradkettiges oder verzweigtes Halogenalkyl, Halogenalkyloxy oder Halogenalkylthio mit jeweils bis zu 4 Kohlenstoffatomen und bis zu 9 Halogenatomen, Halogenalkylendioxy mit 1 oder 2 Kohlenstoffatomen und bis zu 4 Halogenatomen, oder Phenoxy, welches gegebenenfalls die vorausgehend für Aryl und Heteroaryl genannten Substituenten enthält.

**3.** Substituierte Pyrimidylamidoxime gemäß Anspruch 1 der Formel (I), in welcher

- $R^1$ für Wasserstoff, Fluor, Chlor, Brom, Methyl, Ethyl, Trifluormethyl, Chlormethyl, Methoxymethyl, Methoxy, Ethoxy, Difluormethoxy, 2,2,2-Trifluorethoxy, 2-Methoxy-ethoxy, Methylthio, Methylamino, Ethylamino oder Dimethylamino steht,
- $R^2$ für Wasserstoff, Fluor, Chlor, Brom, Methyl, Trifluormethyl oder Methoxy steht,

$R^3$ für Wasserstoff, Fluor, Chlor, Brom, Methyl, Ethyl, Propyl, Trifluormethyl, Chlormethyl, Methoxy, Ethoxy oder Difluormethoxy steht,

A für Methan-1,1-diyl (Methylen), Ethan-1,1-diyl (Ethyliden) oder Ethan-1,2-diyl (Dimethylen) steht, und

Ar für jeweils gegebenenfalls substituiertes Phenyl, Naphthyl, Furyl, Thienyl, Pyrrolyl, Pyrazolyl, Imidazolyl, Triazolyl, Oxazolyl, Benzoxazolyl, Isoxazolyl, Thiazolyl, Benzthiazolyl, Oxadiazolyl, Thiadiazolyl, Pyridyl, Pyrimidyl oder Triazinyl steht, wobei die möglichen Substituenten insbesondere ausgewählt sind aus der Reihe Fluor, Chlor, Brom, Cyano, Phenyl, Methyl, Ethyl, Methoxy, Ethoxy, Methylthio, Ethylthio, Methoxycarbonyl, Ethoxycarbonyl, Trifluormethyl, Trifluormethoxy, Trifluormethylthio, Methylendioxy, Ethylendioxy, Difluormethylendioxy, Trifluormethylendioxy, Tetrafluorethylendioxy, Chlortrifluorethylendioxy, oder Phenoxy, welches gegebenenfalls die vorausgehend für die Aryl- und Heteroarylreste genannten Substituenten enthält.

**4.** Verfahren zur Herstellung von substituierten Pyrimidylamidoximen der allgemeinen Formel (I)

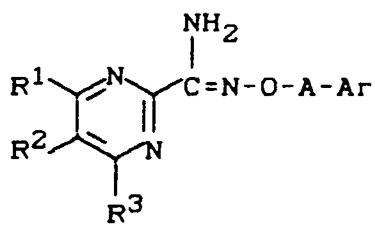

(I)

in welcher

$R^1$, $R^2$ und $R^3$ gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, wobei zwei dieser Reste $R^1$ und $R^2$ oder $R^2$ und $R^3$ auch zusammen für Alkandiyl stehen können,

A für Alkandiyl steht und

Ar für jeweils gegebenenfalls substituiertes Aryl oder Heteroaryl steht,

dadurch gekennzeichnet, daß man Pyrimidylamidoxime der allgemeinen Formel (II)

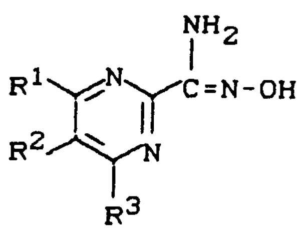

(II)

in welcher

$R^1$, $R^2$ und $R^3$ gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, wobei zwei dieser Reste $R^1$ und $R^2$ oder $R^2$ und $R^3$ auch zusammen für Alkandiyl stehen können,

mit Alkylierungsmitteln der allgemeinen Formel (III)

X-A-Ar (III)

in welcher

A für Alkandiyl steht,

Ar für jeweils gegebenenfalls substituiertes Aryl oder Heteroaryl steht und

X für Halogen steht,

gegebenenfalls in Gegenwart eines Säureakzeptors und gegebenenfalls in Gegenwart eines Verdünnungsmittels umsetzt.

**5.** Schädlingsbekämpfungsmittel, gekennzeichnet durch einen Gehalt an mindestens einem substituiertem Pyrimidylamidoxim der Formel (I) nach den Ansprüchen 1 und 4.

**6.** Verwendung von substituierten Pyrimidylamidoximen der Formel (I) nach den Ansprüchen 1 und 4 zur Bekämpfung von Schädlingen.

**7.** Verfahren zur Bekämpfung von Schädlingen, dadurch gekennzeichnet, daß man mindestens ein Pyrimidylamidoxim der Formel (I) nach den Ansprüchen 1 und 4 auf Schädlinge und/oder ihren Lebensraum einwirken läßt.

**8.** Verfahren zur Herstellung von Schädlingsbekämpfungsmitteln, dadurch gekennzeichnet, daß man mindestens ein Pyrimidylamidoxim der Formel (I) nach den Ansprüchen 1 und 4 mit Streckmitteln und/oder oberflächenaktiven Mitteln vermischt.

**9.** Pyrimidylamidoxime der allgemeinen Formel (II)

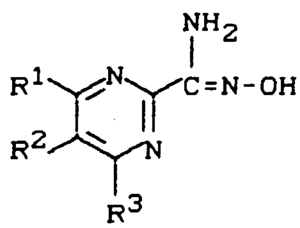

(II)

in welcher

$R^1$, $R^2$ und $R^3$ gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, wobei zwei dieser Reste $R^1$ und $R^2$ oder $R^2$ und $R^3$ auch zusammen für Alkandiyl stehen können,

mit Ausnahme von Pyrimidin-2-amidoxim ($R^1 = R^2 = R^3 = H$).

**10.** Verfahren zur Herstellung von Pyrimidylamidoximen der allgemeinen Formel (II)

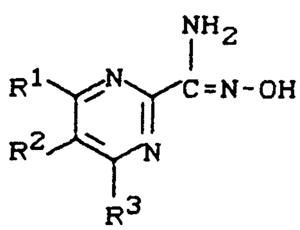

(II)

in welcher

$R^1$, $R^2$ und $R^3$ gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, wobei zwei dieser Reste $R^1$ und $R^2$ oder $R^2$ und $R^3$ auch zusammen für Alkandiyl stehen können,

dadurch gekennzeichnet, daß man Cyanopyrimidine der allgemeinen Formel (IV)

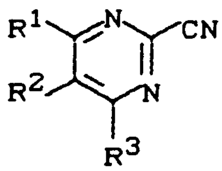

(IV)

in welcher

$R^1$, $R^2$ und $R^3$ die oben angegebene Bedeutung haben,

mit Hydroxylamin oder dessen Säureaddukten, gegebenenfalls in Gegenwart eines Säureakzeptors, und gegebenenfalls in Gegenwart von Verdünnungsmitteln, bei Temperaturen zwischen 20° C und 100° C umsetzt.

11. Verwendung von Pyrimidylamidoximen der Formel (II) gemäß den Ansprüchen 9 und 10 als Ausgangsverbindungen für biologisch hochaktive Stoffe.

**Patentansprüche für folgenden Vertragsstaat : ES**

1. Verfahren zur Herstellung von substituierten Pyrimidylamidoximen der allgemeinen Formel (I)

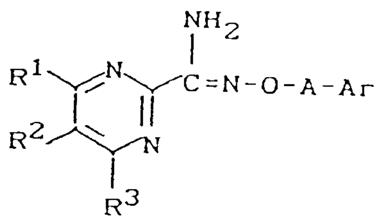

(I)

in welcher

$R^1$, $R^2$ und $R^3$ gleich oder verschieden sind und für Wasserstoff, Halogen, Alkyl, Halogenalkyl, Alkoxyalkyl, Alkoxy, Halogenalkoxy, Alkoxyalyoxy, Alkylthio, Alkylamino oder Dialkylamino stehen, wobei zwei dieser Reste $R^1$ und $R^2$ oder $R^2$ und $R^3$ auch zusammen für Alkandiyl stehen können,

A für Alkandiyl steht und

Ar für jeweils gegebenenfalls substituiertes Aryl oder Heteroaryl steht,

dadurch gekennzeichnet, daß man Pyrimidylamidoxime der allgemeinen Formel (II)

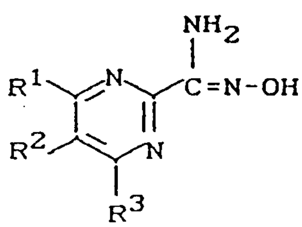

(II)

in welcher

$R^1$, $R^2$ und $R^3$ gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, wobei zwei dieser Reste $R^1$ und $R^2$ oder $R^2$ und $R^3$ auch zusammen für Alkandiyl stehen können,

mit Alkylierungsmitteln der allgemeinen Formel (III)

X-A-Ar (III)

in welcher

A für Alkandiyl steht,

Ar für jeweils gegebenenfalls substituiertes Aryl oder Heteroaryl steht und

X für Halogen steht,

gegebenenfalls in Gegenwart eines Säureakzeptors und gegebenenfalls in Gegenwart eines Verdünnungsmittels umsetzt.

**2.** Schädlingsbekämpfungsmittel, gekennzeichnet durch einen Gehalt an mindestens einem substituiertem Pyrimidylamidoxim der Formel (I) nach dem Anspruch 1.

**3.** Verwendung von substituierten Pyrimidylamidoximen der Formel (I) nach dem Anspruch 1 zur Bekämpfung von Schädlingen.

**4.** Verfahren zur Bekämpfung von Schädlingen, dadurch gekennzeichnet, daß man mindestens ein Pyrimidylamidoxim der Formel (I) nach dem Anspruch 1 auf Schädlinge und/oder ihren Lebensraum einwirken läßt.

**5.** Verfahren zur Herstellung von Schädlingsbekämpfungsmitteln, dadurch gekennzeichnet, daß man mindestens ein Pyrimidylamidoxim der Formel (I) nach dem Anspruch 1 mit Streckmitteln und/oder oberflächenaktiven Mitteln vermischt.

**6.** Verfahren zur Herstellung von Pyrimidylamidoximen der allgemeinen Formel (II)

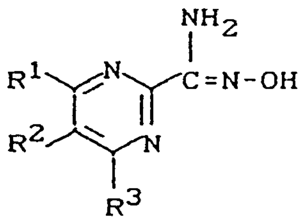

(II)

in welcher

$R^1$, $R^2$ und $R^3$ gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, wobei zwei dieser Reste $R^1$ und $R^2$ oder $R^2$ und $R^3$ auch zusammen für Alkandiyl stehen können,

mit Ausnahme von Pyrimidin-2-amidoxim ($R^1$ = $R^2$ = $R^3$ = H) dadurch gekennzeichnet, daß man Cyanopyrimidine der allgemeinen Formel (IV)

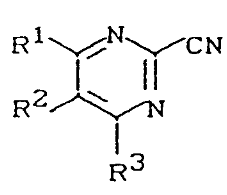

(IV)

in welcher

$R^1$, $R^2$ und $R^3$ die oben angegebene Bedeutung haben,

mit Hydroxylamin oder dessen Säureaddukten, gegebenenfalls in Gegenwart eines Säureakzeptors, und gegebenenfalls in Gegenwart von Verdünnungsmitteln, bei Temperaturen zwischen 20° C und 100° C umsetzt.

**7.** Verwendung von Pyrimidylamidoximen der Formel (II) gemäß dem Anspruch 6 als Ausgangsverbindungen für biologisch hochaktive Stoffe.

Europäisches Patentamt

# EUROPÄISCHER RECHERCHENBERICHT

Nummer der Anmeldung

EP 91 12 0533

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | US-A-4 413 999 (CH. LINDER ET AL.)<br>* Spalte 22; Ansprüche; Beispiel 9 *<br>----- | 1 | C07D239/30<br>C07D239/34<br>C07D239/70<br>C07D401/12<br>C07D403/12<br>C07D405/12<br>A01N43/54 |
| | | | **RECHERCHIERTE SACHGEBIETE (Int. Cl.5 )** |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 17 MAERZ 1992 | FRANCOIS J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)